# EUROPÄISCHE PATENTSCHRIFT

(11) **EP 0 564 622 B1**
(45) Veröffentlichungstag und Bekanntmachung des Hinweises auf die Patenterteilung: **17.01.1996**
(21) Anmeldenummer: 92921842.8
(22) Anmeldetag: 26.10.1992
(51) Int. Cl.: A61F 2/58, A61F 2/80

(54) **ELLBOGEN-PASSTEIL**
ARTIFICIAL ELBOW
COUDE ARTIFICIEL

(30) Priorität: 25.10.1991 DE 4135229
(43) Veröffentlichungstag der Anmeldung: 13.10.1993
(73) Patentinhaber: Otto Bock Orthopädische Industrie Besitz- und Verwaltungs-KG, D-37115 Duderstadt (DE)
(72) Erfinder: GLABISZEWSKI, Richard, D-3408 Duderstadt (DE)
(74) Vertreter: Gramm, Werner, Prof., Dipl.-Ing.
(86) Internationale Anmeldenummer: DE9200891
(87) Internationale Veröffentlichungsnummer: WO9307836

(56) Entgegenhaltungen:
- DE-B- 1 268 782
- DE-C- 415 916
- FR-A- 1 061 971
- GB-A- 182 670
- US-A- 2 033 150
- US-A- 2 537 338
- US-A- 2 812 961
- US-A- 5 007 937

## Beschreibung

Die Erfindung betrifft ein Ellbogen-Paßteil für Oberarmstümpfe, wobei eine Ellbogenkugel durch eine Gelenkachse mit dem als hohles Kunststoffteil ausgebildeten Unterarm verbunden und in verschiedenen Beugestellungen durch eine Rastenfeststellung verriegelbar ist, während die proximale Verbindung zum Oberarmschaft über einen Eingußring hergestellt und zur einstellbaren Drehung des Unterarmes ein als Ringlager ausgebildetes Oberarm-Drehgelenk vorgesehen ist.

Eine derartige Ausführungsform läßt sich der US-A 2,537,338 entnehmen.

Die FR-A 1.061.971 offenbart ein Ellbogen-Paßteil, bei dem die eingangs erwähnte Rastenfeststellung als Zugsperre ausgebildet ist, die in eine einen Abschnitt der Gelenkachse bildende Drehverbindung integriert ist.

Die GB-A 182,670 offenbart ein Ellbogen-Paßteil für Oberarmstümpfe mit einem als Ringlager ausgebildeten Drehgelenk.

Der Erfindung liegt die Aufgabe zugrunde, das eingangs beschriebene Ellbogen-Paßteil so zu gestalten, daß es sich für alle Oberarmstumpflängen eignet.

Ausgehend von dem eingangs beschriebenen Ellbogen-Paßteil wird diese Aufgabe erfindungsgemäß durch folgende Merkmale gelöst:
a) Die Rastenfeststellung ist eine außerhalb der Ellbogenkugel angeordnete, in eine einen Abschnitt der Gelenkachse bildende Drehverbindung integrierte Zugsperre;
b) das Oberarm-Drehgelenk umfaßt einen in die Ellbogenkugel eingesetzten und mit dieser drehfest verbundenen Lagerring, der einen Ringsteg aufweist, der von einem Bremsring umgriffen wird, der mit dem Eingußring drehfest verbunden ist, der gegenüber dem Lagerring um 180° verdrehbar ist;
c) der Bremsring ist von einem Bremsstück beaufschlagbar, das gegen eine sich über 180 Umfangsgrad erstreckende, den Verschwenkbereich des Eingußringes definierende Anfasung einer Ringkante des Bremsringes anliegt;
d) das Bremsstück ist über eine von außen zugängliche Verschraubung radial gegen den Bremsring verspannbar;
e) der Lagerring übergreift die Ellbogenkugel mit einem Ringflansch, auf dem sich der Eingußring abstützt.

Die erfindungsgemäßen Maßnahmen ermöglichen die Versorgung von Patienten beliebiger Stumpflänge bis Ellbogenexartikulation mit nur einem Drehgelenk. Denn durch die Ausbildung des Oberarm-Drehgelenkes als Ringlager anstelle des bisher verwendeten Planlagers erhält man den erforderlichen Raum für nahezu beliebige Stumpflänge. Durch die außen angeordnete Zugsperre ist die Geometrie für lange Stümpfe gewährleistet; eine konstruktive Anpassung an unterschiedliche Stumpflängen ist nicht erforderlich.

Weitere Merkmale sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen
- **Figur 1** -: ein Ellbogen-Paßteil in Seitenansicht und zum Teil im Längsschnitt;
- **Figur 2** -: die Darstellung gemäß **Figur 1** um 90° gedreht und im Längsschnitt;
- **Figur 3** -: in vergrößertem Maßstab ein Detail der **Figur 2**;
- **Figur 4** -: in Explosionsdarstellung eine Baugruppe einer Zugsperre;
- **Figur 5** -: in Draufsicht eine an eine Gelenkplatine angelenkte Zugsperre;
- **Figur 6** -: in einer Explosionsdarstellung eine weitere Baugruppe der Zugsperre gemäß **Figur 5**;

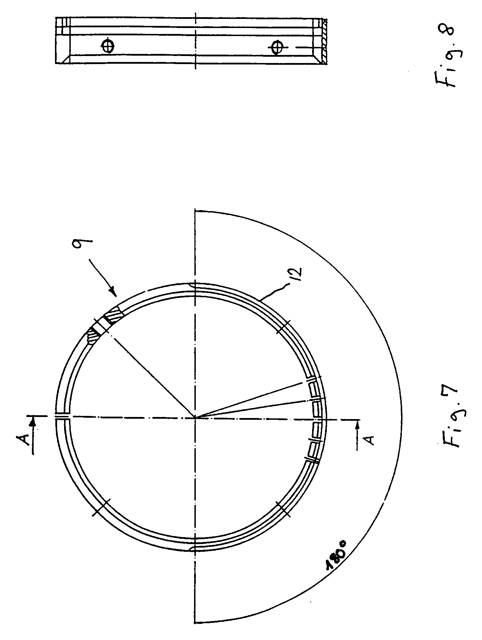

- **Figur 7** -: in Draufsicht einen Bremsring und
- **Figur 8** -: einen Schnitt gemäß der Linie A - A in **Figur 7.**

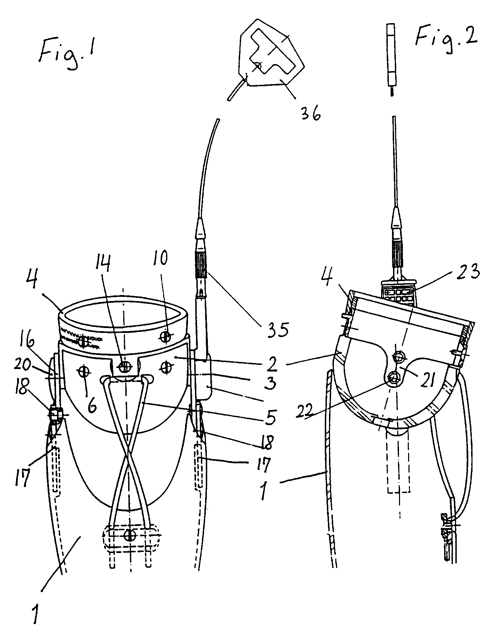

Das in den **Figuren 1** und **2** dargestellte Ellbogen-Paßteil besteht im wesentlichen aus einem als hohles Kunststoffteil ausgebildeten Unterarm 1, an dessem oberen Ende eine Ellbogenkugel 2 um eine Gelenkachse 3 verschwenkbar gelagert und in verschiedenen Beugestellungen durch eine Rastenfeststellung verriegelbar ist. Für die proximale Verbindung zu einem nicht dargestellten Oberarmschaft ist ein Eingußring 4 vorgesehen, der Teil eines Oberarm-Drehgelenkes zur einstellbaren Drehung des Unterarmes 1 ist. Bei frei schwingendem Unterarm 1 wird die Streckung durch einen vorderen Anschlag aus Perlondraht 5 begrenzt, der einerseits an der Ellbogenkugel 2 und andererseits am Unterarm 1 befestigt ist.

Das Oberarm-Drehgelenk umfaßt einen in die Ellbogenkugel 2 eingesetzten und mit dieser über Schrauben 6 drehfest verbundenen Lagerring 7, der einen Ringsteg 8 aufweist, der von einem Bremsring 9 umgriffen wird, der mit dem Eingußring 4 über Schrauben 10 drehfest verbunden ist. Der Bremsring 9 ist geschlitzt ausgebildet und läßt sich so auf den genannten Ringsteg 8 aufsetzten. Die der Ellbogenkugel 2 zugewandte Ringkante 11 des Bremsringes 9 weist über 180 Umfangsgrad eine Anfasung 12 auf, gegen die ein z. B. aus einem Buntmetall bestehendes Bremsstück 13 über eine von außen zugängliche Verschraubung 14 radial verspannbar ist. Durch Verstellen der Verschraubung 14 läßt sich die auf den Bremsring 9 ausgeübte Bremskraft einstellen.

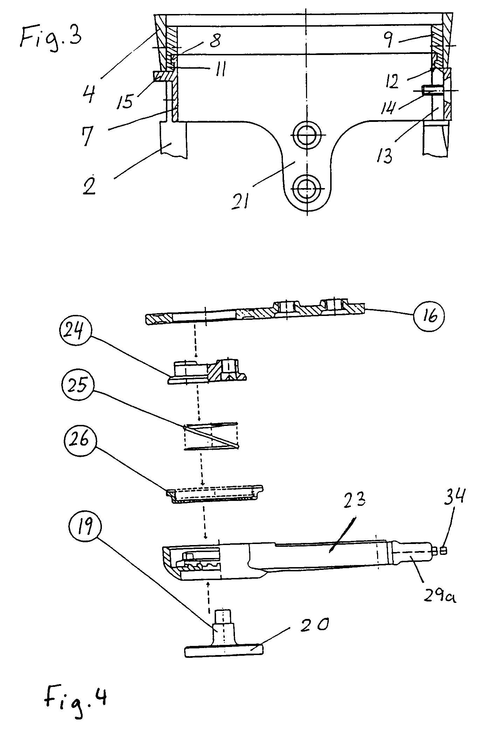

Der Lagerring 7 übergreift die Ellbogenkugel 2 mit einem Ringflansch 15, auf dem sich der Eingußring 4 abstützt (siehe insbesondere **Figuren 2** und **3**), der sich über die Anfasung 12 des Bremsringes 9 und somit um 180° verdrehen läßt.

Die Gelenkachse 3 wird durch zwei miteinander fluchtende Drehverbindungen gebildet, über die die Ellbogenkugel 2 zwischen zwei Gelenkplatinen 16 verschwenkbar gelagert ist, die mit seitlichen in den Unterarm 1 integrierten Schienen 17 über Schrauben 18 fest verbunden sind. Jede Drehverbindung weist einen Gelenkzapfen 19 auf, der mit einem Kopf 20 außen an der zugeordneten Gelenkplatine 16 (rastenlose Drehverbindung) bzw. an einem Gehäuse 23 der Rastenfeststellung anliegt und mit seinem anderen Ende mit einer Fahne 21 des Lagerringes 7 verschraubt ist (Schrauben 22).

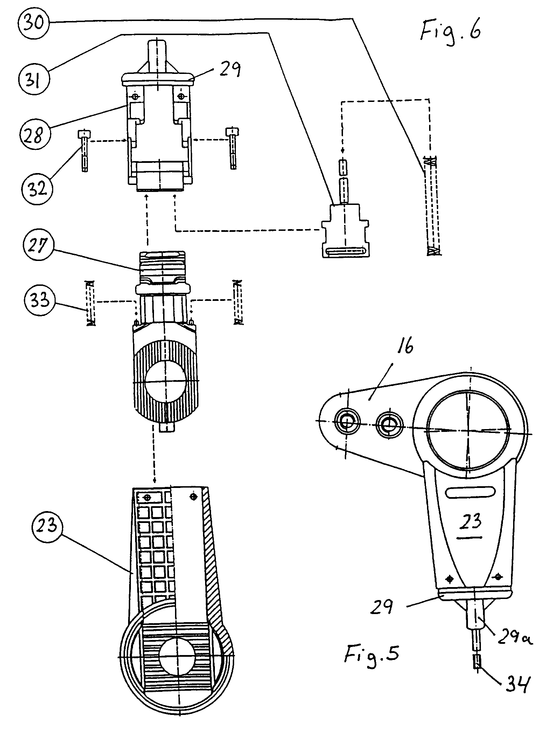

Außerhalb der Ellbogenkugel 2 ist in die eine die Gelenkachse 3 bildende Drehverbindung eine Rastenfeststellung in Form einer Zugsperre integriert. Diese ist mit dem Gehäuse 23 auf dem einen Gelenkzapfen 19 verdrehbar an der zugeordneten Gelenkplatine 16 gelagert, wie es **Figur 5** zeigt. Gemäß der Explosionsdarstellung in **Figur 4** schließen das Gehäuse 23 sowie die Gelenkplatine 16 zwischen sich einen Mitnehmer 24, eine Druckfeder 25, eine Rastscheibe 26 sowie eine Schaltfeder 27 (siehe **Figur 6**) ein.

In das Gehäuse 23 ist eine Lade 28 eingezogen, die das Gehäuse 23 mit einem Flansch 29 abschließt und einen gegen die Wirkung einer Druckfeder 30 verschiebbaren Keil 31 umfaßt, der Schaltklinken 32 betätigt, die von Druckfedern 33 beaufschlagt sind. Dem Keil 31 ist die Schaltfeder 27 zugeordnet.

Der Keil 31 weist an seinem einen Ende einen Gewindestift 34 auf, der durch den Flansch 29 und einen an diesen angeformten Stutzen 29a der Lade 28 hindurchgesteckt ist. Auf diesen Gewindestift 34 ist ein in den **Figuren 1** und **2** dargestelltes Zugseil 35 aufgeschraubt, an dessen freien Ende ein Bügel 36 zur manuellen Betätigung der Zugsperre befestigt ist.

Durch Anziehen des Bügels 36 läßt sich die Zugsperre entriegeln; die Ellbogenkugel 2 ist zwischen den Gelenkplatinen 16 gegenüber dem Unterarm 1 frei verschwenkbar. Durch erneutes Anziehen des Bügels 36 erfolgt eine Verrastung der Ellbogenkugel 2 in der gewünschten Beugestellung, wobei z. B. achtzehn Raststellungen vorgesehen sein können.

## Patentansprüche

1. Ellbogen-Paßteil für Oberarmstümpfe, wobei eine Ellbogenkugel (2) durch eine Gelenkachse (3) mit dem als hohles Kunststoffteil ausgebildeten Unterarm (1) verbunden und in verschiedenen Beugestellungen durch eine Rastenfest stellung verriegelbar ist, während die proximale Verbindung zum Oberarmschaft über einen Eingußring (4) hergestellt und zur einstellbaren Drehung des Unterarmes (1) ein als Ringlager ausgebildetes Oberarm-Drehgelenk (7 - 15) vorgesehen ist, **gekennzeichnet durch** folgende Merkmale:
a) die Rastenfeststellung ist eine außerhalb der Ellbogen kugel (2) angeordnete, in eine einen Abschnitt der Gelenkachse (3) bildende Drehverbindung (19 - 22) integrierte Zugsperre (23 - 36);
b) das Oberarm-Drehgelenk (7 - 15) umfaßt einen in die Ellbogenkugel (2) eingesetzten und mit dieser drehfest verbundenen Lagerring (7), der einen Ringsteg (8) aufweist, der von einem Bremsring (9) umgriffen wird, der mit dem Eingußring (4) drehfest verbunden ist, der gegenüber dem Lagerring (7) um 180° verdrehbar ist;
c) der Bremsring (9) ist von einem Bremsstück (13) beauf schlagbar, das gegen eine sich über 180 Umfangsgrad er streckende, den Verschwenkbereich des Eingußringes (4) definierende Anfasung (12) einer Ringkante (11) des Bremsringes (9) anliegt;
d) das Bremsstück (13) ist über eine von außen zugängliche Verschraubung (14) radial gegen den Bremsring (9) verspannbar;
e) der Lagerring (7) übergreift die Ellbogenkugel (2) mit einem Ringflansch (15), auf dem sich der Eingußring (4) abstützt.

2. Ellbogen-Paßteil nach Anspruch 1, **dadurch gekennzeichnet**, daß der Bremsring (9) geschlitzt ist.

3. Ellbogen-Paßteil nach einem der vorhergehenden Ansprüche, **dadurch gekennzeichnet**, daß die Gelenkachse (3) durch zwei miteinander fluchtende Drehverbindungen (19 - 22) gebildet wird, über die die Ellbogenkugel (2) zwischen zwei mit dem Unterarm (1) verbundenen Gelenkplatinen (16) verschwenkbar gelagert ist.

4. Ellbogen-Paßteil nach Anspruch 3, **dadurch gekennzeichnet**, daß jede Drehverbindung (19 - 22) einen Gelenkzapfen (19) aufweist, der mit seinem innen liegenden Ende mit einer Fahne (21) des Lagerringes (7) verschraubt ist.

5. Ellbogen-Paßteil nach einem der vorigen Ansprüche, **dadurch gekennzeichnet**, daß die Zugsperre (23 - 36) mit einem Gehäuse (23) verdrehbar auf dem einen Drehzapfen (19) gelagert ist.

6. Ellbogen-Paßteil nach Anspruch 5, **dadurch gekennzeichnet**, daß das genannte Gehäuse (23) zusammen mit der zugeordneten Gelenkplatine (16) zwischen sich einen Mitnehmer (24), eine Druckfeder (25), eine Rastscheibe (26) sowie eine Schaltfeder (27) einschließen.

7. Ellbogen-Paßteil nach Anspruch 5 oder 6, **dadurch gekennzeichnet**, daß in dem Gehäuse (23) gegen die Wirkung einer Druckfeder (30) ein Schaltklinken (32) betätigender Keil (31) verschiebbar gelagert ist, der an seinem einen Ende einen aus dem Gehäuse (23) herausragenden Gewindestift (34) trägt, auf den ein Zugseil (35) aufschraubbar ist.

## Claims

1. An elbow adapter part for upper arm stumps, an elbow ball (2) being connected by an articulated axis (3) to the lower arm (1), which is constructed as a hollow plastics part, and the elbow ball (2) being lockable in various bent positions by a latch fixing, while the proximal connection to the upper arm shaft is made by way of a cast-in ring (4) and an upper arm rotary articulation (7 - 15) is provided, constructed as a ring bearing, for the adjustable rotation of the lower arm (1), characterized by the following features:
a) the latch fixing is a traction lock (23 - 26) which is arranged outside the elbow ball (2) and is integrated in a rotary connection (19 - 22) forming a section of the articulation axis (3);
b) the upper arm rotary articulation (7 - 15) comprises a bearing ring (7) which is inserted in the elbow ball (2) and connected non-rotatably thereto, which has an annular web (8) surrounded by a braking ring (9) which is non-rotatably connected to the cast-in ring (4), the latter being rotatable by 180° with respect to the bearing ring (7);
c) the braking ring (9) can be acted upon by a braking piece (13) which bears against a chamfer (12) of an annular edge (11) of the braking ring (9), this chamfer (12) extending peripherally over 180 degrees and defining the pivotal range of the cast-in ring (4);
d) the braking piece (13) can be tensioned radially against the braking ring (9) by way of an externally accessible screw (14);
e) the bearing ring (7) reaches over the elbow ball (2) by means of an annular flange (15) on which the cast-in ring (4) is supported.

2. An elbow adapter part according to Claim 1, characterized in that the braking ring (9) is slotted.

3. An elbow adapter part according to one of the preceding claims, characterized in that the articulation axis (3) is formed by two mutually flush rotary connections (19 - 22) by way of which the elbow ball (2) is pivotally mounted between two articulation plates (16) connected to the lower arm (1).

4. An elbow adapter part according to Claim 3, characterized in that each rotary connection (19 - 22) has an articulation journal (19) which is screwed by means of its inside end to a tab (21) on the bearing ring (7).

5. An elbow adapter part according to one of the preceding claims, characterized in that the traction lock (23 - 36) is mounted on the one rotary journal (19) in a manner rotatable with a housing (23).

6. An elbow adapter part according to Claim 5, characterized in that the said housing (23) and the associated articulation plate (16) together enclose between them an entrainer (24), a pressure spring (25), a latching disc (26) and a switch spring (27).

7. An elbow adapter part according to Claim 5 or 6, characterized in that a wedge (31) actuating switching catches (32) is mounted displaceably in the housing (23) in opposition to the action of a pressure spring (30), this wedge (31) bearing on its one end a threaded pin (34) which projects out of the housing (23) and onto which a traction cable (35) can be screwed.

## Revendications

1. Pièce d'adaptation de coude pour moignon de bras, avec une sphère de coude (2) reliée par un axe d'articulation (3) à l'avant-bras (1), présentant la forme d'une pièce creuse en matière synthétique et pouvant être immobilisée en différentes positions de flexion par un système d'immobilisation à encliquetage, tandis que la liaison proximale au bras est réalisée par une bague coulée (4), une articulation à rotation (7 - 15) par rapport au bras étant prévue pour la rotation réglable de l'avant-bras (1), caractérisée par les particularités ci-dessous:
a) le système d'immobilisation par encliquetage est un frein de traction (23 - 36) disposé à l'extérieur de la sphère du coude (2) et intégré dans une liaison à rotation (19 - 22) formant une partie de l'axe d'articulation (3);
b) l'articulation à rotation par rapport au bras (7 - 15) comprend une bague de palier (7) insérée dans la sphère du coude (2) et solidaire des rotations de celle-ci, et présentant une aile annulaire (8) qui est entourée par une bague de freinage (9) solidaire des rotations de la bague coulée (4) qui peut tourner de 180° par rapport à la bague de palier (7);
c) une pièce de freinage (13), qui repose contre un chanfrein (12) d'un bord annulaire (11) de la bague de freinage (9), s'étendant sur une plage de 180° et définissant la plage de pivotement de la bague de coulée (4) agit sur la bague de freinage (9);
d) la pièce de freinage (13) peut être serrée radialement contre la bague de freinage (9) par l'intermédiaire d'un vissage (14) accessible de l'extérieur;
e) la bague de palier (7) chevauche la sphère du coude (2) par une bride annulaire (15) sur laquelle s'appuie la bague coulée (4).

2. Pièce d'adaptation de coude selon la revendication 2, caractérisée en ce que la bague de freinage (9) est fendue.

3. Pièce d'adaptation de coude selon l'une des revendications précédentes, caractérisée en ce que l'axe d'articulation (3) est formé par deux liaisons à rotation (19 - 22) alignées l'une sur l'autre, par l'intermédiaire desquelles la sphère du coude (2) est maintenue à inclinaison entre deux plaques d'articulation (16) reliées à l'avant-bras (1).

4. Pièce d'adaptation de coude selon la revendication 3, caractérisée en ce que chaque liaison à rotation (19 - 22) présente un pivot d'articulation (19) qui est vissé à une aile (21) de la bague de palier (7) par son extrémité située à l'intérieur.

5. Pièce d'adaptation de coude selon l'une quelconque des revendications précédentes, caractérisée en ce que le verrouillage à traction (23 - 36) est maintenu à rotation avec un boîtier (23) sur un des pivots de rotation (19).

6. Pièce d'adaptation de coude selon la revendication 5, caractérisée en ce qu'ensemble, ledit boîtier (23) et la plaque d'articulation (16) associée enferment entre eux un dispositif d'entraînement (24), un ressort de compression (25), un disque d'encliquetage (26) ainsi qu'un ressort d'inversion (27).

7. Pièce d'adaptation de coude selon la revendication 5 ou 6, caractérisée en ce qu'une clavette (31) actionnant un cliquet d'inversion (32) est maintenue à glissement dans le boîtier (23), en opposition à l'action d'un ressort de compression (30), la clavette (31) portant à une de ses extrémités une tige filetée (34) débordant du boîtier (23) et sur laquelle un câble de traction (35) peut être vissé.
